Europäisches Patentamt

European Patent Office

Office européen des brevets

(19)

(11) Publication number: **0 131 974**
**A1**

## (12) EUROPEAN PATENT APPLICATION

(21) Application number: 84200762.7

(22) Date of filing: 25.05.84

(51) Int. Cl.⁴: **G 01 N 33/53**

(30) Priority: 08.06.83 NL 8302038

(43) Date of publication of application:
23.01.85 Bulletin 85/4

(84) Designated Contracting States:
AT BE CH DE FR GB IT LI LU NL SE

(71) Applicant: AKZO N.V.
Velperweg 76
NL-6824 BM Arnhem(NL)

(72) Inventor: Kuypers, Leonardus Paulus Clemens
Gelissenstraat 24
NL-5344 JV Oss(NL)

(72) Inventor: Wolters, Gerrit
Mozartlaan 4
NL-5343 EL Oss(NL)

(74) Representative: Douma, Anno Dominicus et al,
Postbus 20
NL-5340 BH Oss(NL)

(54) Determination of delta-antigens in body fluids.

(57) The present invention concerns a method for detection of the HBV associated S-antigen in a sample comprising a first incubation with carrier-bonded anti-HBs and a second incubation with anti-δ in the presence of a detergent.

Also claimed are test kits for carrying out this method and an immunochemical reagent comprising anti-HBs and anti-δ conjointly bonded to a carrier.

1

# DETERMINATION OF DELTA-ANTIGENS IN BODY FLUIDS

The invention relates to a method for the qualitative and/or quantitative detection of the delta-antigen related to hepatitis B in a sample of a body fluid, and to test kits for carrying out such a method.

In the 1940's it was discovered that many cases of jaundice are caused by viral pathogens – the hepatitis viruses. Hepatitis A and hepatitis B viruses have been identified. In addition, so-called non-A, non-B hepatitis is recognized, of which no pathogen has been identified with certainty. For making a correct diagnosis and for a treatment of jaundice appropriate to this diagnosis it is of course necessary to identify the possible pathogen. Differentiation between the types of hepatitis virus is first of all possible on the basis of the clinical course of the illness. A more trust-worthy identification is however provided by immunochemical characterization. Detection of antigens of the known types of hepatitis virus and of the antibodies against these is currently carried out on a large scale; not only in the blood of patients suspected to be infected with hepatitis but also in the blood of potential carriers of the disease which do not exhibit any clinical symptoms of the disease, in particular of donors.

Donor blood is screened as a matter of routine for the presence of substances characteristic of the hepatitis virus and in particular the presence of antigens of hepatitis B virus (HBV) and generally also of antibodies against these. This has led to the fact that, for example in the U.S.A., the percentage of receivers who contracted a hepatitis infection through blood transfusion has dropped from 17.9% to 5.9%. In order constantly to repress viral hepatitis further it is necessary to include the detection of as many as possible of the substances characterizing the viruses in the routine screening of patients and non-patients.

Recently, a new type of antigen related to HBV, the so-called delta-antigen ($\delta$-Ag), and an immunoglobulin directed against it (anti-$\delta$) have been discovered. The start of this discovery was the histochemical detection (with the aid of immunofluorescence) of this new antigen in liver cells, obtained by biopsy, of patients with chronic liver disease which were positive for substances characteristic of hepatitis B virus. Probably we are dealing with an antigen of an RNA virus which, for a number of its vital life functions, depends on the hepatitis B virus. The occurrence of $\delta$-Ag in the liver and of anti-$\delta$ in the blood hence is regarded as a symptom of a super-infection which further reinforces the life-endangering character of a chronic HBV infection.

The $\delta$-infection leads to frequently very serious acute hepatitis which in certain cases can occur very suddenly and violently and is then fatal.

Retrospective investigations have shown that a large number of hepatitis infections which previously were regarded as hepatitis non-A, non-B cases were in reality $\delta$-infections. Other investigations have shown that about 7% of the total population infected with HBV also is infected with $\delta$-Ag. However, the HBV-infected patients who regularly receive blood or products obtained from blood form a group with a much greater risk of $\delta$-infection. Thus about 50% of the investigated HBV-infected haemophiliacs in Italy, Germany and the U.S.A. (who are administered coagulation factors) appear also to be infected with $\delta$-Ag.

In chronic hepatitis B patients, where $\delta$-Ag has been found in liver biopsies, this has always been paired with the occurrence of anti-$\delta$ in the blood, and this last parameter is also generally treated as an indicator of a $\delta$-infection. Though, the presence of immunoglobulins, directed against $\delta$-Ag does not in all cases indicate an active $\delta$-infection, and the only reliable parameter for this is the presence of $\delta$-Ag itself in the blood. A method for the unambigious detection of $\delta$-Ag in the blood is however not available, but is of the very greatest importance for the unequivocal diagnosis of the infection and for the complete screening of donor blood for substances characteristic of $\delta$-infection.

The invention now provides a method whereby, surprisingly, sensitive detection of $\delta$-Ag in body fluids is possible.

The method according to the invention is characterized in that a sample of the body fluid to be investigated is incubated with carrier-bonded immunoglobulins directed against hepatitis B surface antigen (hereafter referred to as anti-HBs), thereafter the solid phase is separated from the liquid phase, the solid phase is incubated, in the presence of a detergent and of immunoglobulins directed against $\delta$-Ag (anti-$\delta$) and thereafter the possible bonding of $\delta$-Ag to anti-$\delta$ is determined, giving a qualitative and/or quantitative measure of the presence of $\delta$-Ag in the sample.

The carrier for anti-HBs may have any shape or size which permits the separation of the carrier-bonded and non-carrier-bonded phases from one another and may be manufactured of any material which suites for this purpose. Anti-HBs can be bonded directly or indirectly to the carrier. Suitable carriers are, for example, test tubes, wells of microtitration strips or plates, and rods, beads or discs made of glass, plastics or naturally occurring substances.

The detergent used in the method according to the invention can be, for example, sodium dodecylsulphate, sodium dodecyl-N-sarcosinate, cetyltrimethylammonium bromide, dodecylpyrimidinium chloride, palmitoyl-lysolecithin, dodecyl-N-betaine, polyoxyethylene alcohols, polyoxyethylene-iso-alcohols, polyoxyethylene-p-t-octyl-phenols, polyoxyethylene nonylphenols, polyoxyethylene esters of fatty acids, polyoxyethylene sorbitol esters, sodium dodecylsulphonate, tetradecylammonium bromide and saponins. The non-ionic detergents are preferred. Very suitable non-ionic detergents are polyoxyethylene derivatives, including Bry, Triton, Nonidet-P-40 and Tween.

The detection of the bond of δ-Ag to anti-δ can be carried out by any method customary in immunochemistry. For example, it is possible to use, for this purpose, an agglutination reaction or a so-called sandwich reaction.

In the determination of δ-Ag with the aid of an agglutination reaction, δ-Ag can be incubated with anti-δ bonded to particles. The possible presence of δ-Ag will then lead to agglutination of the particles, and this can be detected qualitatively or quantitatively. In this method the particles used can be, for example, erythrocytes, latex particles, dye sol particles or sol particles of metals or metal compounds.

In the determination of δ-Ag with the aid of a sandwich reaction, δ-Ag is be incubated with carried-bonded anti-δ as well as with anti-δ bonded to a labelling agent. The joint bonding of both anti-δ reagents to one δ-Ag will lead to the bonding of the labelling agent to the carrier, and the detection of carrier-bonded labelling agent provides a measure of the bonding of δ-Ag to anti-δ, and thus of the δ-Ag present in the sample. Just as in the case of anti-HBs, the carrier used can be manufactured of any suitable material, and can be of any shape and size which makes it possible to separate the carrier-bonded and non-carrier-bonded phases from one another. The carrier-bonded anti-HBs and anti-δ, which can be used in the method according to the invention, can each be bonded to different carriers and be brought into contact simultaneously or independently of one another with the sample to be investigated, or can be conjointly bonded to one and the same carrier.

This last possibility will in general constitute the method of determination which is simplest to carry out. In such a method, all conventional labelling agents can be used for the detection of $\delta$-Ag, such as enzymes, radioactive atoms or compounds, fluorescent substances, coloured compounds, dye sols, sols of metals or metal compounds, and the like.

The invention also relates to a test kit which contains at least some of the components required for carrying out the method according to the invention. Hence, a test kit at least contains anti-HBs bonded to a carrier, as well as anti-$\delta$. Anti-$\delta$ can, for example, be present in a particle-bonded form in the test kit, but on the other hand a test kit can contain both carrier-bonded anti-$\delta$ and anti-$\delta$ bonded to a labelling agent. In the last-mentioned test kit, anti-HBs and anti-$\delta$ can be present either conjointly on one carrier or on separate carriers. As carrier or carriers it is possible to use, for example, test tubes, microtitration plates or strips, or rods, beads or discs manufactured from, for example, glass, plastic or a naturally occurring substance. As labelling agent, a test kit according to the invention can, for example, contain: enzymes, radioisotopes or radioisotope-containing compounds, fluorescent or coloured compounds or sols of dyes, metals or metal compounds. If desired, a test kit can also contain auxiliaries for carrying out the determination, such as auxiliaries for the detection of the labelling agents (for example a substrate for enzymes), rinsing buffers and/or detergents. As particles bonded to anti-$\delta$ a test kit according to the invention can, for example, contain erythrocytes;

latex particles or sol particles of dyes, metals or metal compounds.

The invention will be further explained with the aid of the examples which follow.

## Example I
### Detection of δ-Ag in serum in polystyrene tubes coated with anti-HBs and anti-δ

A. Methods

A.1. Coating of polystyrene tubes.

Polystyrene tubes were coated on the inside with anti-HBs and anti-δ by incubation with a mixture of human immunoglobulins against HBsAg and δ-Ag in bicarbonate buffer of pH 9.0 for 24 hours at 20 °C. Thereafter the tubes were washed with 0.15 mol/litre phosphate buffer of pH 7.2.

A.2. Coupling of the anti-δ to horseradish
    peroxidase.

A conjugate of immunoglobulins against δ-Ag and horseradish peroxidase (HRP) was prepared according to the method of Nakane & Kawaoi (J.Histochem.Cytochem. 1974, 22, 1084-1091).

A.3. Incubation of coated polystyrene tubes with serum.

The coated polystyrene tubes were incubated for 2 hours at 37 °C with 1.0 ml of the serum to be examined, in a series of increasing dilutions with 0.15 mol/litre phosphate buffer of pH 7.2. Thereafter, the tubes were sucked empty, washed three times with 2 ml of 0.5 mol/litre phosphate buffer of pH 7.2, and sucked empty.

A.4. Incubation with detergent.

The tubes obtained under A.3. were incubated for 2 hours at 37 °C with 1.0 ml of a solution of Nonidet-P-40 in 0.15 mol/litre phosphate buffer of pH 7.2. Thereafter the tubes were sucked empty, washed three times with 2 ml of O.15 mol/litre phosphate buffer of pH 7.2, and sucked empty.

A.5. Incubation with an anti- $\mathscr{E}$-HRP-conjugate.

To each of the tubes obtained under A.4. was added 1 ml of the conjugate obtained under A.2., diluted in O.15 mol/litre of phosphate buffer of pH 7.2. After incubation for 1 hour at 37 °C, the tubes were sucked empty, washed three times with 2 ml of O.15 mol/litre phosphate buffer of pH 7.2, and again sucked empty.

A.6. Incubation with substrate for HRP.

Thereafter, 1 ml of a mixture of O.4 mg/ml of ortho-phenylenediamine and O.4 mg/ml of urea peroxide in O.15 mol/litre sodium acetate of pH 5.0 was added to the tubes. After 30 minutes' incubation, the reaction was stopped by adding 1 ml of 2 mol/litre sulphuric acid solution.

A.7. Measurements.

The absorptions of the solutions thus obtained in the polystyrene tubes were measured in a photometer at a wavelength of 492 nm.

B. Results.

The results of the absorption measurements according to the method described above, using samples of serums of patients with chronic HBV infection and samples of human control serums in various dilutions are shown in Table 1.

Table 1

| Dilutions | Undiluted | 1:2 | 1:4 | 1:8 |
|---|---|---|---|---|
| | Absorptions at 492 nm | | | |
| Human serum <br> HBV-infected <br> $\delta$-Ag positive | 1.873 | 1.063 | 0.682 | 0.479 |
| HBV-infected <br> $\delta$-Ag negative | 0.288 | 0.277 | 0.263 | 0.280 |
| Control serum <br> $\delta$-Ag negative | 0.293 | 0.279 | 0.281 | 0.269 |
| Control serum <br> $\delta$-Ag positive | 0.501 | | | |

Example II

Determination of $\delta$-Ag before and after $\delta$-infection

Before and after the appearance of clinical hepatitis, blood samples were taken from a patient suffering from a clinical hepatitis, and serums were prepared from these samples. These serums were tested in a system wherein anti-HBs coated onto the inside of microtitration plate wells and anti-$\delta$ coated onto rods were used.

A. Methods.

A.1. Coating of polystyrene microtitration plates.

The wells of a polystyrene microtitration plate were coated on the inside with anti-HBs by incubating them for 24 hours at room temperature with human immunoglobulins directed against HBsAg in bicarbonate buffer of pH 9.0 and subsequently washing them with 0.15 mol/litre phosphate buffer of pH 7.2.

A.2. Coating of polystyrene rods.

Polystyrene rods, of such dimensions that they fit into the wells of the microtitration plates, were coated with immunoglobulins directed against $\delta$-Ag in a manner analogous to the procedure described under A.1.

A.3. Coupling of anti-$\delta$ with horseradish peroxidase.

This conjugate was prepared in the manner described in Example I.A.2.

A.4. Incubation of the microtitration plate with the serums to be investigated.

0.1 ml of the serums to be investigated was pipetted into the wells of the microtitration plate and incubated for 1 hour at 37 $^{\circ}$C. Thereafter the wells were sucked empty and washed five times with 0.3 ml of phosphate buffer of pH 7.2.

A.5. Incubation with detergents.

Thereafter, 0.1 ml of a solution of 3 g/litre of Nonidet-P-40 in phosphate buffer of pH 7.2 was introduced into each of the wells, and a polystyrene rod coated with anti-$\delta$, obtained under A.2., was dipped into each well. After 1 hour's incubation at 37 $^{\circ}$C, the rods were taken out of the liquid and washed by dipping them five times in succession in a phosphate buffer of pH 7.2, a fresh portion of the buffer being used for each dipping.

A.6. Incubation with anti-$\delta$-HRP conjugate.

The polystyrene rods thus treated were subsequently dipped into a solution of the conjugate obtained under A.3., and after incubation for 1 hour at 37 $^{\circ}$C they were washed as described under A.5.

A.7. Incubation with substrate for HRP.

Thereafter the rods were placed in the wells of a microtitration plate which contained 0.2 ml of a mixture of 0.4 mg/ml of ortho-phenylenediamine and 0.4 mg/ml of urea peroxide in a buffer of pH 5.0. After incubation for 30 minutes, the rods were removed and 0.1 ml of 2 mol/litre sulphuric acid were added to the liquid in the wells.

A.8. Measurements.

The absorptions of these solutions were measured in a photometer at a wavelength of 492 nm $(A_{492})$.

B. Results.

The absorptions of the serums, examined in accordance with the method described above, of a patient in various phases of a $\delta$-infection are reproduced in Table 2.

## Table 2
### $\delta$-Ag measurement in various stages of $\delta$-infection

| Time | $A_{492}$ |
|---|---|
| 1 month before infection | 0.305 |
| During infection | 1.347 |
| 1 month after infection | 0.759 |
| 3 months after infection | 0.315 |
| 7 months after infection | 0.320 |
| 2 years after infection | 0.317 |
| Negative control serum | 0.331 |
| $\delta$-Ag positive control serum | 0.572 |

12

### Example III
### Determination of $\delta$-Ag in serums of a group of HBV patients and in a group of control serums.

The serum of 20 healthy persons and of 12 chronic HBV patients (including 6 patients with acute hepatitis) were investigated for the presence of HBsAg, HBeAg, Dane particles and $\delta$-Ag; in the HBV patients, the liver cells obtained by biopsy were moreover examined for the presence of $\delta$-Ag.

A. Methods

A.1. Delta-antigen determination in liver biopsy samples.

The presence of $\delta$-Ag in liver cells obtained by biopsy was revealed by means of immunofluorescence in accordance with the method of Rizzetto et al. (Gut, 1977, 18, 997-1003).

A.2. Detection of HBsAg in serum.

For the detection of HBsAg in serum, use was made of an ELISA method according to G. Wolters et al. (J.Clin.Pathol., 1976, 29, 873-879).

A.3. Detection of HBeAg in serum.

HBeAg in serum was determined with the aid of an ELISA method according to M. van de Waart et al. (J.Med.Virol., 1979, 3, 43-49).

A.4. Dane particles in serum.

For the detection of Dane particles, serum was examined with the aid of an electron microscope.

A.5. Detection of $\delta$-Ag in serum.

$\delta$-Ag in serum was determined according to the method described in Example II.

B. Results.

The results of the various determinations in the serums examined are reproduced in Table 3. These results show that the test for $\delta$-Ag in serum according to the invention does not react with human serum proteins, HBeAg, HBcAg which occurs in Dane particles, or Dane particles themselves, and also not with HBsAg. The test for $\delta$-Ag in serum was positive in patients with acute hepatitis, $\delta$-Ag being detectably present in the liver cells.

Table 3

$\delta$-Ag in serums of HBV patients and in control serums

| | | $\delta$-Ag in liver cells | HBeAg in serum | Dane particles in serum | $\delta$-Ag in serum, absorption at 492 nm | | |
|---|---|---|---|---|---|---|---|
| 12 HBV patients | 6 acute patients | 3 pos. | 3 neg. | 3 neg. | 1.902; | >2.000; | >2.000 |
| | | 3 neg. | 3 pos. | 3 pos. | 0.315; | 0.321; | 0.317 |
| | 6 non-acute HBV carriers | 6 neg. | 3 neg. | 3 neg. | 0.318; | 0.312; | 0.311 |
| | | | 3 pos. | 3 pos. | 0.306; | 0.304; | 0.299 |
| 20 controls | | - | 20 neg. | 20 neg. | average ($\pm$ S.D.) 0.312 ($\pm$ 0.013) | | |

Example IV

Test kit

A test kit which is used for carrying out the $\delta$-Ag determination according to Example I comprises the following components:

- ampoules with freeze-dried human immunoglobulins directed against $\delta$-Ag and labelled with horseradish peroxidase;
- polystyrene tubes, packaged in aluminium laminate and coated on the inside with immunoglobulins directed against $\delta$-Ag and against HBsAg;
- bottles containing phosphate buffer of pH 7.2;
- a bottle containing a solution of Nonidet-P-40;
- a bottle with effervescent tablets which contain ortho-phenylenediamine;
- urea peroxide tablets in blister strips;

- sodium acetate buffer;
- a flask with $\delta$-Ag-negative control serum;
- a flask with $\delta$-Ag-positive control serum;
- instructions for carrying out the test.

1

## CLAIMS

1.      A method for the qualitative and/or quantitative detection of the delta-antigen related to hepatitis B in a sample of a body fluid, characterized in that the sample to be examined is incubated with carrier-bonded immunoglobulins, directed against hepatitis B surface-antigen, thereafter the solid phase is separated from the liquid phase, the solid phase is incubated, in the presence of a detergent and of immunoglobulins directed against the delta-antigen, and thereafter the possible bonding of delta-antigen to immunoglobulins, directed against this antigen, is determined, which bonding gives a qualitative and/or quantitative measure of the presence of a delta-antigen in the sample.

2.      Method according to claim 1, characterized in that the incubation with immunoglobulins, directed against the delta-antigen, is carried out with carrier-bonded immunoglobulins and with immunoglobulins bonded to a labelling agent, and thereafter the possible bonding of delta-antigen to immunoglobulins, directed against this antigen, is determined by determining the bonding of the labelling agent to the carrier-bonded immunoglobulins, directed against delta-antigen.

3.      Method according to claim 2, characterized in that a carrier is used to which the immunoglobulins, directed against hepatitis B surface antigen and against delta-antigen are bonded conjointly.

4.      Method according to claim 2 or 3, characterized in that an enzyme is used as the labelling agent.

5.      Method according to claim 1, characterized in that the incubation with immunoglobulins, directed against the delta-antigen, is carrier out with immunoglobulins bonded to particles.

6.      Method according to claim 5, characterized in that erythrocytes, latex particles, dye sols or metal sols are used as particles.

7.      Method according to claim 1-6, characterized in that the detergent is a non-ionic detergent.

8.      Method according to claim 7, characterized in that the non-ionic detergent is a polyoxyethylene derivative.

9.      Test kit for carrying out the method according to claim 1, characterized in that this kit comprises at least the following:
   - carrier-bonded immunoglobulins directed against hepatitis B surface antigens; and
   - immunoglobulins directed against delta-antigen.

10.    Test kit according to claim 9, characterized in that this kit comprises at least the following:
- a carrier to which are bonded immunoglobulins, directed against hepatitis B surface antigen and against delta-antigen; and
- immunoglobulins, bonded to a labelling agent, directed against delta-antigen.

11.    Test kit according to claim 9, characterized in that this kit comprises at least the following·
- immunoglobulins directed against hepatitis B surface antigen, bonded to a first carrier;
- immunoglobulins directed against delta-antigen, bonded to a second carrier; and
- immunoglobulins directed against delta-antigen, bonded to a labelling agent.

12.    Test kit according to claim 10 or 11, characterized in that this kit contains an enzyme as the labelling agent.

13.    Test kit according to claim 12, characterized in that this kit additionally contains a substrate for the enzyme.

14.    Test kit according to claim 9, characterized in that this kit comprises at least the following:
- immunoglobulins directed against hepatitis B surface antigen, bonded to a carrier; and
- immunoglobulins directed against delta-antigen, bonded to particles.

15.    Test kit according to claim 14, characterized in that this kit contains erythrocytes, latex particles, dye sols or metal sols as particles.

16.    Test kit according to claim 9-15, characterized in that it additionally comprises a detergent.

17.    Immunochemical test reagent comprising a carrier and bonded there to immunoglobulins directed against delta-antigen and directed against hepatitis B surface antigen.

## European Patent Office

# EUROPEAN SEARCH REPORT

0131974

Application number

EP 84 20 0762

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-3 116 882 (GEORGETOWN UNIVERSITY, WASHINGTON D.C.) * Claims 1-3; page 14, lines 5-22 * | 1,2 | G 01 N 33/53 |
| A | JOURNAL OF CLINICAL MICROBIOLOGY, vol. 14, no. 2, August 1981, pages 173-177; O. CRIVELLI et al.: "Enzyme-linked immunosorbent assay for detection of antibody to the hepatitis B surface antigen-associated delta antigen" * Whole article * | 1,2 | |
| A | GB-A-2 051 357 (ABBOTT LABORATORIES) * Whole document * | 1-3,9-15 | |
| A | GB-A-1 442 546 (ABBOTT LABORATORIES) * Whole document * | 1,5,6 | TECHNICAL FIELDS SEARCHED (Int. Cl.4) G 01 N C 12 N A 61 K |
| A | EP-A-0 024 174 (VENTREX LABORATORIES) * Whole document * | 10,11 | |

The present search report has been drawn up for all claims

| Place of search | Date of completion of the search | Examiner |
|---|---|---|
| THE HAGUE | 14-09-1984 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82